# EUROPEAN PATENT APPLICATION

(11) **EP 3 539 446 A1**
(43) Date of publication of application: **18.09.2019**
(21) Application number: 18161752.3
(22) Date of filing: 14.03.2018
(51) Int. Cl.: A61B 1/00, A61B 1/07, G02B 23/24

(54) **A TIP PART FOR A VISION DEVICE**

(71) Applicant: Ambu A/S, 2750 Ballerup (DK)
(72) Inventor: SØRENSEN, Morten, 2750 Ballerup (DK); JENSEN, Thomas Bachgaard, 1721 Copenhagen V (DK); JACOBSEN, Morten, 2970 Hørsholm (DK)
(74) Representative: AWA Denmark A/S

(57) **Abstract**

A tip part (5) for a vision device. The tip part (5) comprises a housing (6, 7) adapted for accommodating a vision receptor (10). At least one light guide (15) of a transparent material having a predetermined length is arranged between at least one first light reception end (16) comprising an end face (16) with an essentially plane surface perpendicular to the longitudinal direction adapted for receiving light from a light source and at least one second light emission end (17) adapted to emit light.

The light guide (15) has varying cross-sectional area along the predetermined length, so that the predetermined length comprises an expanding sector and/or a narrowing sector in which the light guide has a varying cross-sectional area along the predetermined length.

## Description

The present invention relates to vision devices such as but not limited to endotracheal tubes and endoscopes, more specifically to a tip part of such a vision device and a vision device such as an endoscope with such a tip part.

Vision device such as endoscopes are well known for visually inspecting inaccessible places such as human body cavities. Typically, the endoscope comprises an elongated insertion tube with a handle at the proximal end as seen from the operator and visual inspections means, such as a built in camera, at the distal end of the elongated insertion tube. Electrical wiring for the camera and other electronics such as LED lighting accommodated in the tip part at the distal end run along the inside of the elongated insertion tube from the handle to the tip part. Instead of using cameras, endoscopes may also be fibre-optic, in which case the optical fibres run along inside of the elongated insertion tube to the tip part.

In order to be able to manoeuvre the endoscope inside the body cavity, the distal end of the endoscope may comprise a bending section with increased flexibility, e.g. a number of articulated segments of which the tip part forms the distalmost segment. This is typically done by tensioning or slacking pull wires also running along the inside of the elongated insertion tube from the tip part through the remainder of articulated segments to a control mechanism of the handle. Furthermore, a working channel may run along the inside of the insertion tube from the handle to the tip part, e.g. allowing liquid to be removed from the body cavity or allowing the insertion of surgical instruments or the like into the body cavity.

As the name indicates, endoscopes, are used for seeing inside things, such as lungs or other human body cavities of a patient. Modern endoscopes are therefore typically equipped with a least one camera or similar image capturing device at the distal tip of the endoscope. Provided that sufficient light is present, this allows the operator to see where the endoscope is steered and to set the target of interest once the tip has been advanced thereto. This therefore normally requires illumination of the area in front of the distal tip of the endoscope, in particular the field of vision of the camera(s). One known way of achieving such illumination is to provide the above mentioned LED lighting using one or more Light Emitting Diodes (LEDs) in the tip of the endoscope, as e.g. mentioned in WO2014/106511 disclosing a disposable endoscope. The light distribution in the emitted light from a light source such as an optical fibre or an LED is, however, not optimal as regards the field of vision of a vision receptor such as an image sensor, a video camera or an optical fibre. In particular an LED may spread the emitted light over a wide angle. If light is emitted at an angle so wide that it illuminates objects outside the field of vision of the vision receptor, it is largely wasted. Also, in many body cavities, in particular tubular ones, objects in the periphery of the field of vision will often be closer than those in the centre. Consequently, they will be stronger illuminated than those in the centre, which in turn leads to overexposure of the image at the periphery and underexposure in the centre where the object of interest is often likely to be.

When, as in the present invention, the insertion tube of the endoscope is intended to be inserted into a human body cavity, the insertion tube furthermore needs to be sealed in a watertight manner. This is in particular the case for the distal tip part because it accommodates the camera, LED(s) and other delicate electronics, prone to malfunction or destruction if exposed to humidity.

One known way of sealing the tip part of an endoscope is disclosed in WO2010/066790. In this document a transparent monolithic housing is formed around the electronics and working channel by placing the electronics and the tube forming the working channel in a mould of transparent material, such as silicone. A transparent UV curable resin is then inserted from the bottom of the mould to avoid bubbles to form in the transparent resin. Because the resin rises slowly from the bottom, the air is slowly expelled from top of the mould, without any risk of air bubbles being trapped in the mould. The resin is the cured using UV irradiation through the transparent mould to form the monolithic housing. However, forming a monolithic housing in this way has some drawbacks. One is that it is a somewhat slow process. Another is that it can be difficult to position and maintain the components precisely in position during the insertion of the resin. Thus the camera or LEDs may be off-set sideways or a thin transparent layer may inadvertently be created in front of the camera and/or LEDs, thereby reducing the imaging qualities of the tip part.

Based on this prior art it is the object of the present invention to provide a tip part of an endoscope which overcomes at least some of the above problems.

According to a first aspect of the present invention this object is achieved by a tip part for a vision device, such as an endoscope, said tip part comprising a housing part adapted for accommodating a vision receptor, at least one light guide of a transparent material having a predetermined length in a longitudinal direction between at least one first light reception end adapted for receiving light from a light source and at least one second light emission end adapted to emit light, wherein the predetermined length comprises an expanding sector and/or a narrowing sector in which the light guide has a varying cross-sectional area along the predetermined length, wherein, in said expanding sector, the cross-sectional area is monotonously increasing in the direction from the at least one light reception end towards the at least one light emission end and in said narrowing sector, the cross-sectional area is monotonously decreasing, and wherein the first light reception end comprises an end face with an essentially plane surface.

In this way it becomes possible to control the light emission characteristics from the tip of the endoscope, in particular so as to obtain a desired light distribution profile, matching e.g. the field of vision of the vision receptor, while at the same time ensuring a good coupling of light from the light source into the light guide.

According to a first preferred embodiment the predetermined length further comprises one or more sectors of constant cross-sectional area. This is advantageous when the predetermined length exceeds the necessary length of the expanding and/or narrowing sectors. In this way angles may still be chosen of the expanding and/or narrowing sectors which ensure total internal reflection in these sectors.

Thus, according to a further preferred embodiment, said monotonously increasing and/or decreasing cross-sectional area changes in such a way along the length of said at least one sector that total internal reflection of light from the light source is provided.

According to another preferred embodiment of the present invention, said at least one sector with the monotonously increasing cross-sectional area comprises a truncated pyramid. Having a linearly expanding cross-section provides good control over the total internal reflection properties of the light guide, in turn aiding in providing the above mentioned desired light distribution profile.

According to another preferred embodiment, the cross-sectional area has, along at least a part of the predetermined length, the shape of a rectangle. A rectangular shape is preferable because the vision receptor will normally comprise a rectangular field of vision. By also having a cross sectional shape of the light guide, the desired light distribution profile may be made to match at least partially the field of vision of the vision receptor. Thus, less light is wasted.

According to a specifically preferred embodiment, the rectangle has rounded corners. Thereby, disturbances and deviations from the optimally desired light distribution profile may be minimized.

According to a further preferred embodiment, at least the sector with monotonously increasing cross-sectional area of said light guide is surrounded by air. Having air around the light guide, and in particular around the sector with monotonously increasing cross-sectional area, provides good total internal reflection in the light guide and accordingly low light losses.

According to an alternative embodiment, however, at least a part of the light guide is cladded with a cladding. Providing a cladding gives good control over the total internal reflection, and secures that the desired properties are not ruined by ingress of pollutants, e.g. sealing material or glue.

According to another preferred embodiment, the light reception end is adapted to engage an LED. Adapting the right reception end to engage the LED, e.g. by matching surface areas, ensures good light transmission properties across the interface between the LED and the light guide, e.g. avoiding reflections. Furthermore, when the light reception end is so adapted that no glue or the like is necessary any deterioration of phosphorescent material on the LED that could be caused by the glue is avoided.

According to a further preferred embodiment, the light reception end is adapted to engage a light fibre. Adapting the right reception end to engage the light fibre, e.g. by providing the plane surface in the bottom of a bore, ensures good light transmission properties across the interface between the light fibre and the light guide, e.g. avoiding reflections. Furthermore, when the light reception end is so adapted that no glue or the like is necessary any undesired reflections that could be caused by the glue are avoided.

According to yet another preferred embodiment the essentially plane surface of the end face is perpendicular to the longitudinal direction. This further improves the control of the light and the good coupling thereof into the light guide.

According to yet another preferred embodiment, the transparent material comprises polycarbonate. Polycarbonate is advantageous in that it is has good optical properties, is easy to mould, and has low material costs.

According of yet a further preferred embodiment, the light guide forms an integral part of the housing. By integrating the light guide in the housing, it becomes possible to provide a sealed front end of the tip part.

According to a second aspect of the present invention, the object of the invention is solved by providing an endoscope with a tip as described above. any one of the preceding claims.

The invention will now be described in greater detail based on nonlimiting exemplary embodiments and with reference to the drawings, on which:
Fig. 1 shows an endoscope in which the present invention is implemented,
Fig. 2 shows an isometric view of tip part for an endoscope in accordance with the present invention,
Fig. 3 shows an exploded view of the housing of the tip part of Fig. 1 with light guides in according to one embodiment of the invention,
Fig. 4 shows a cross-section of the tip part taken along the line IV-IV in Fig. 2 together with parts of attached tubes,
Fig. 5 shows a cross-section of the tip part taken along the line V-V in Fig. 2 together with parts of a bending section, and
Fig. 6, 7 and 8 schematically show different embodiments of the light guide according to the invention.

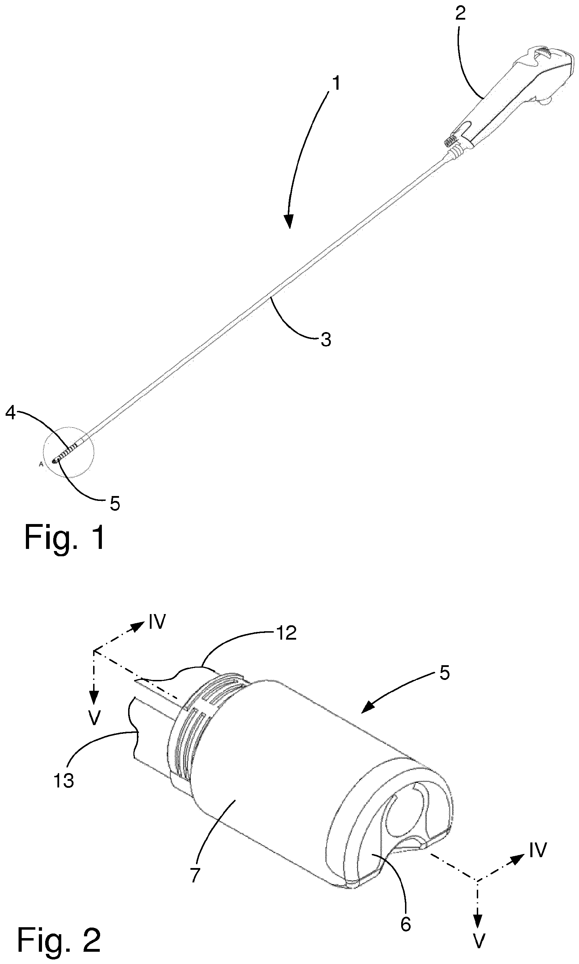

Turning first to Fig. 1, an endoscope 1 exemplifying the vision device according to the invention is shown. The endoscope 1 comprises a handle 2 at the proximal end, an insertion tube 3 extending towards the distal end where it comprises an articulated bending section 4, which as the most distal segment has a distal tip part 5 according to the invention. Though omitted for illustration purposes the articulated bending section 4 will normally be covered by a suitable sleeve, connected at least at its own distal end to the distal tip part 5, e.g. by means of an adhesive. This as such is conventional and e.g. known from the aforementioned WO2014/106511. The endoscope 1 of the present invention is intended as a disposable endoscope to be thrown away after use and low manufacturing costs is therefore an important issue.

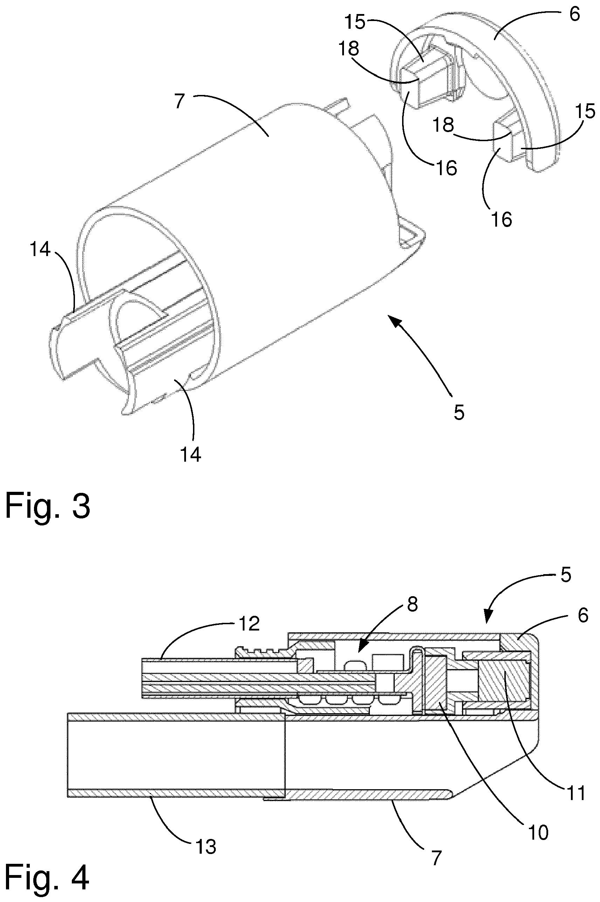

As best seen in Figs. 2 and 3 illustrating the preferred embodiment, the distal tip part 5 comprises two housing parts. A first housing part 6 made of a suitable transparent material, and a second housing part 7 optimized for other properties such as opacity, softness, elasticity, and/or good adhesive bonding. Though shown as two parts in the exploded view of Fig. 3, the housing need not be assembled from two parts but could be manufactured as a single item of a single transparent material or as a single item of two materials fused or bonded together in the moulding process.

As best seen in Fig. 4 an electronics module 8 is accommodated in the distal tip part. In the illustrated embodiment the electronics module comprises a light source in the form of a pair of LEDs 9 and a vision receptor such as a video camera 10 or an imaging chip. Possibly with an additional lens assembly 11 arranged in front of the video camera 10 or imaging chip.

Electrical wiring to the electronics module 8 and/or optical fibres for illumination or image capture, if used instead of LEDs and video camera, are lead in to the housing via a conduit 12 and sealed using a suitable sealing material (not shown). For the purposes of this description, however, reference is made only to LEDs 9 and video cameras 10, as the skilled person will understand the interchangeability with the above-mentioned alternatives.

Also connected to the housing and sealed is a tube 13 forming part of the working channel of the endoscope 1. In the illustrated embodiment, the tube 13 is connected via a pair of protrusions between which the tube 13 is held, possibly with the aid of an adhesive.

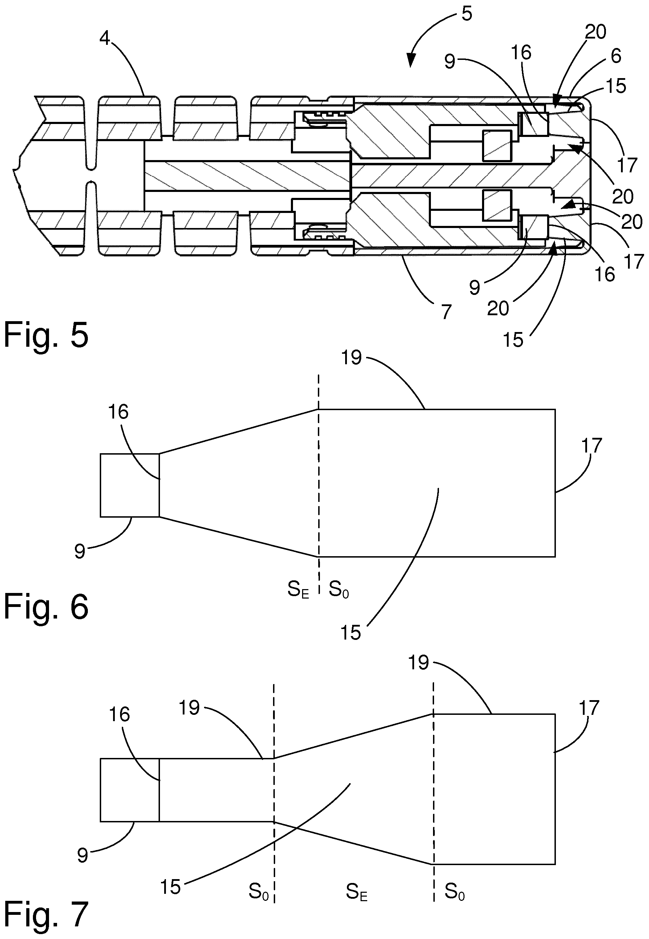

To achieve a desired illumination characteristics or light distribution profile from the light source, i.e. the pair of LEDs 9, the LEDs are located in a retracted manner within the housing, and at least one light guide 15 is associated to the LEDs. At the proximal end, the light guide 15 thus comprises a first light receiving end 16 adapted to receive light from the LED, e.g. by having a plane surface matching that of the LED or a bore in which the LED is inserted. At the distal end, the light guide comprises a second light emission end 17 adapted to emit the light received from the LED 9. In one preferred embodiment, the light guide forms an integral part of the housing as can best be seen from Figs. 3 and 5. That is to say the light guide 15 is moulded integrally with the transparent housing part 6. Alternatively, they may be made as separate elements, as indicated in Figs. 6, 7 and 8, interposed between the LEDs and the transparent housing part 6.

The light guides 15 are configured to utilize total internal reflection TIR to provide the desired illumination characteristics or light distribution profile from the light source, this configuration may involve index of refraction of the material, transparency, length, variation of the cross-sectional area, geometry of the cross-section, cladding etc.

Since video cameras 10 are generally adapted for capturing a rectangular image it has been realized that using light guides 15 with a generally rectangular cross-section is advantageous. As compared to circular ones, more light can be directed towards the corners. Also, potentially less light will be wasted by not illuminating areas outside the rectangular field of vision of the video camera. This applies even if a pair of LEDs 9 is used, and even if the shape of the rectangular cross-section does not match the aspect ratio of the video camera 10. It has been found, however, that using rectangular cross-sections with rounded corners 18, as can be seen in Fig. 3, is advantageous as it gives better control over the light distribution in the corners of the field of vision of the video camera 10. In particular artefacts produced by multiple reflections in sharp corners are avoided. Preferably, the rectangular cross-section as is present along the entire length of the light guide 15, be it with the rounded corners 18 or not. Though less preferred, the use of other cross-sectional shapes, in particular elliptical or circular, but also polygons with or without rounded corners are not excluded.

To utilize best possibly the total internal reflection, the cross-sectional area of the light guide 15 varies along the predetermined length. As can be seen from Figs. 6 to 8, the predetermined length comprises a number of sectors, indicated by S_{E}, S₀ and S_{N}, indicating sectors with expanding, constant, and narrowing cross-sectional areas, respectively. More specifically it varies in a way where the cross-sectional area in a sector is either constant, monotonously increasing or monotonously decreasing from the at least one light reception end 16 towards the at least one light emission end 17. In Fig. 3 where the area of cross-section monotonously increasing sector increases linearly the sector thus comprises a truncated pyramid. If the cross-section was different, analogous shapes would apply, e.g. a truncated cone for a circular cross-section. It should be emphasized that the monotonous increase need not be linear.

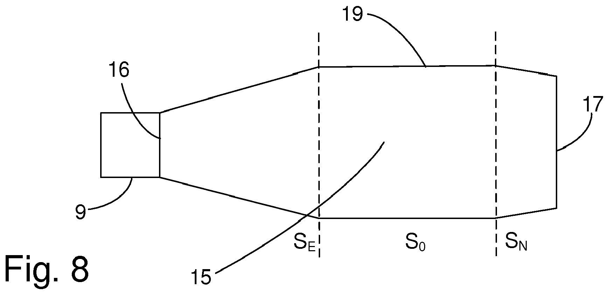

If a sector So with a constant area 19 is also used, this sector 19 could be arranged on any side of the sector with the monotonously increasing cross-sectional area, be it proximal as in Fig. 6, distal or both, as in Fig. 7. It could also be arranged somewhere in the middle as indicated in Fig. 8. If total internal reflection is present in all sectors it is of less importance where the monotonously increasing cross-sectional area is located. However, if there is a cladding there is a risk that there is not total internal reflection. It may therefore be preferred that the increase in area starts immediately from the LEDs which changes the angle of incidence of light impinging on the surface, thereby ensuring total internal reflection of more light towards the light emission end. Thus the light from the LEDs get more parallel or collimated and the angle over which the light is emitted becomes narrower and matches the field of vision of the video camera better. If the collimation is narrower than desired, as it would typically be if the light source is the exit aperture of a light fibre, it may be necessary to provide light scattering features at the exit end of the light guide, such as a chamfering of the edges. Thus, as can be seen in Fig. 8, there is a sector S_{N} towards the light emission end 17, where the cross-sectional area monotonously decreases. Like the increase mentioned above, this decrease is preferably selected to that total internal reflection is ensured.

Other configurations of constant and monotonously increasing areas are not excluded. It should also be noted that the truncated pyramids of Fig. 3 may be implemented as separate elements, and conversely the shapes described in conjunction with Figs. 6,7 and 8 may be formed integrally with the transparent housing part 6. The use of one or more constant area sectors So, may allow the predetermined length to be adapted to other structural features such as the length of the lens assembly 11, the location of the LEDs on a circuit board carrying the video camera 10 and other components of the electronics section 8.

To ensure good total internal reflection in order to keep the light within the light guide and/or control the exit angle from the distal end 17 it is preferred to surround at least the sector S_{E} with monotonously increasing cross-sectional area or the sector S_{N} with monotonously decreasing cross-sectional area of the light guide is surrounded by air as illustrated by the cavities 20 in Fig 5. Alternatively, a cladding, e.g. a glue, with a predetermined index of refraction may be used. A cladding may be advantageous in cases where the components are not arranged in a pre-fabricated housing, but rather moulded-in in housing material after assembly, because in such cases it may be difficult to ensure the existence of the air filled cavities 20 around the light guides 15.

As for the transparency and the index of refraction of the transparent material 6 the transparent material 6 may comprise polycarbonate, which has suitable properties and may be injection moulded for efficient manufacture. If no cladding is present, i.e. the polycarbonate is surrounded by air the minimum angle of incidence of light ensuring total internal reflection is 39 degrees. Other transparent materials will result in other minimum angles, as will cladding.

## Claims

1. A tip part for a vision device, such as an endoscope said tip part comprising
a housing part adapted for accommodating a vision receptor,
at least one light guide of a transparent material having a predetermined length in a longitudinal direction between at least one first light reception end adapted for receiving light from a light source and at least one second light emission end adapted to emit light, wherein
the predetermined length comprises
an expanding sector and/or a narrowing sector in which the light guide has a varying cross-sectional area along the predetermined length,
wherein, in said expanding sector, the cross-sectional area is monotonously increasing in the direction from the at least one light reception end towards the at least one light emission and in said narrowing sector, the cross-sectional area is monotonously decreasing, and wherein the first light reception end comprises an end face with an essentially plane surface.

2. A tip part according to claim 1, wherein the predetermined length further comprises one or more sectors of constant cross-sectional area.

3. A tip part according to any one of the preceding claims, wherein said monotonously increasing and/or decreasing cross-sectional area changes in such a way along the length of said at least one sector that total internal reflection of light from the light source is provided.

4. A tip part according to any one of the preceding claims, wherein said at least one sector with the monotonously increasing and/or decreasing cross-sectional area comprises a truncated pyramid.

5. A tip part according to any one of the preceding claims, wherein the cross-sectional area has, along at least a part of the predetermined length, the shape of a rectangle.

6. A tip part according to claim 5, wherein the rectangle has rounded corners.

7. A tip part according to any one of the preceding claims wherein at least the sector with monotonously increasing and/or decreasing cross-sectional area of said light guide is surrounded by air.

8. A tip part according to any one of claims 1 to 7, wherein at least a part of the light guide is cladded with a cladding.

9. A tip part according to any one of the preceding claims, wherein the light reception end is adapted to engage an LED.

10. A tip part according to any one of claims 1 to 8, wherein the light reception end is adapted to engage a light fibre.

11. A tip part according to any one of the preceding claims, wherein the essentially plane surface of the end face is perpendicular to the longitudinal direction.

12. A tip part according to any one of the preceding claims, wherein the transparent material comprises polycarbonate.

13. A tip part according to any one of the preceding claims, wherein the light guide forms an integral part of the housing.

14. A vision device, such as an endoscope, with a tip according to any one of the preceding claims.
